Europäisches Patentamt

European Patent Office

Office européen des brevets

(11) Publication number: **0 066 465**
**B1**

(12) **EUROPEAN PATENT SPECIFICATION**

(45) Date of publication of patent specification: **26.02.86**

(51) Int. Cl.⁴: **A 61 B 10/00**

(21) Application number: **82302764.4**

(22) Date of filing: **28.05.82**

(54) **Biopsic forceps.**

(30) Priority: **03.06.81 JP 82328/81 u**

(43) Date of publication of application:
**08.12.82 Bulletin 82/49**

(45) Publication of the grant of the patent:
**26.02.86 Bulletin 86/09**

(84) Designated Contracting States:
**AT BE CH DE FR GB IT LI NL SE**

(56) References cited:
**EP-A-0 065 054**
**FR-A-2 355 521**
**FR-A-2 479 680**
**FR-A-2 479 680**
**GB-A-1 069 398**
**GB-A-1 215 439**
**GB-A-2 022 421**

(73) Proprietor: **OLYMPUS OPTICAL CO., LTD.**
**43-2, 2-chome, Hatagaya Shibuya-ku**
**Tokyo 151 (JP)**

(72) Inventor: **Hirayama, Shozo**
**No. 43-13, Sandamachi 3-chome**
**Hachioji-shi Tokyo (JP)**

(74) Representative: **Kennedy, Victor Kenneth et al**
**G.F. REDFERN & CO. Marlborough Lodge 14**
**Farncombe Road**
**Worthing West Sussex BN11 2BT (GB)**

Courier Press, Leamington Spa, England.

# Description

This invention relates to biopsic forceps for taking structural samples within body cavities when inserted through forceps channels of endoscopes or inserted directly into incised body cavities, and more particularly to improved biopsic forceps so constructed that even if the handles are operated so as to cause cutting blades to mesh with each other with a strong force, the drive shaft will be neither bent nor broken.

In recent developments in medical endoscopy, not only is the endoscope inserted into a body cavity to observe a part to be inspected (affected part) but a forceps may be inserted into a body cavity directly or through a forceps channel of the endoscope and operated from outside the body cavity to obtain a biopsic structural specimen for inspection, e.g. from an affected part.

Biopsic forceps of various forms are known for taking structural samples from within body cavities. With regard to the Contracting States CH, DE, GB, and LI, EP—A—0 065 054 describes a biopsic forceps, being comprised in the state of the art according to Art. 54(3), and having a shaft on which a pair of elongate resilient holding pieces are provided at the tip so as to separate and open in the form of V from each other when caused to project from a pipe in which said shaft is slidably fitted, mutually opposed cutting blades being provided at the respective free ends of the pair of holding pieces so that, when the holding pieces project out of the pipe said pair of holding pieces separate the cutting blades and when the holding pieces are retracted into the pipe said pair of holding pieces close to mesh the cutting blades with each other, said shaft being supported at the rear of said pipe by the end of one of a pair of handles having respective finger grips and pivoted to open and close about a fulcrum, said pipe being supported at the rear by the end of the other of said pair of handles, so that when the handles are operated said pipe moves axially forward or rearward on a straight line defined by the axis of said shaft, said pair of handles being pivoted so that when the finger grips are pushed apart said ends of the handles come together, and said shaft (22) being adjustably clamped at the rear of the pipe to the end of its handle. However, in a biopsic forceps wherein a pair of resilient holding pieces are provided at the tip of a shaft slidably inserted through a pipe, so as to separate and open in the form of V from each other when made to emerge, respective opposed cutting blades being provided on these holding pieces so that, when the above mentioned holding pieces are moved to project out of the pipe, they will be separated from each other to open the cutting blades, and when the holding pieces are retracted into the pipe they will be closed to mesh the cutting blades with each other. The structure of a part to be inspected (affected part) may be hard, so that when the handles are strongly urged inwardly a link intersecting the fulcrum and shaft will press the pipe so strongly that the pipe will be pushed up with the pivoting point of the pipe and link as a fulcrum, and as a result the shaft inserted through the pipe will be subjected to an unreasonable bending force, and may be bent or broken, which is most undesirable.

One object of the present invention is to provide a biopsic forceps wherein, even in such cases that a structural specimen of a part to be inspected (affected part) is exceptionally hard, and requires severe force to be applied so as to strongly retract the holding pieces into the pipe, the pipe and shaft will only be subjected to significant force in the axial direction, and there will be no unreasonable force in a bending direction, and therefore will be neither bent nor broken and the above mentioned pipe and shaft may be supported directly by the handles without using such connecting member as a link so as to be able to reduce the number of component parts.

According to the present invention there is provided a biopsic forceps having a shaft on which a pair of elongate resilient holding pieces are provided at the tip so as to separate and open in the form of V from each other when caused to project from a pipe in which said shaft is slidably fitted, mutually opposed cutting blades being pivoted at the respective free ends of the pair of holding pieces so that, when the holding pieces project out of the pipe said pair of holding pieces separate the cutting blades and when the holding pieces are retracted into the pipe said pair of holding pieces close to mesh the cutting blades with each other, said shaft being supported at the rear of said pipe by the end of one of a pair of handles having respective finger grips and pivoted to open and close about a central fulcrum, said pipe being supported at the rear by the end of the other of said pair of handles, so that when the handles are operated said pipe moves axially forward or rearward on a straight line defined by the axis of said shaft, said pair of handles being pivoted by an off-set pin so that when the finger grips are pushed apart said ends of the handles come together, characterised in that the shaft is adjustably clamped at the rear of the pipe to the end of its handle by an off-set clamping means and that the pipe is slidably supported in a U-shaped groove at the end of its handle by an engaging part of a tube provided at the end of the pipe and having incised surfaces to positively locate the pipe axially with respect to the end of its handle whilst permitting freedom of movement in the direction of the longitudinal axis of the handle.

An embodiment of the invention will now be described with reference to the drawings, in which:—

Figure 1 is an elevation of a conventionally constructed forceps showing holding pieces separated in the form of a V;

Figure 2 is an elevation of the conventional forceps shown in Figure 1 with its handles closed by a strong force to cause the holding pieces to be retracted into the pipe, which is then bent up as shown;

Figure 3 is an exploded view of a biopsic forceps constructed in accordance with the present invention;

Figure 4 is an elevation showing the holding pieces of Figure 3 separated to form a V; and

Figure 5 is a perspective view showing the cutting blades meshed with each other when the holding pieces of Figure 3 are retracted into the pipe.

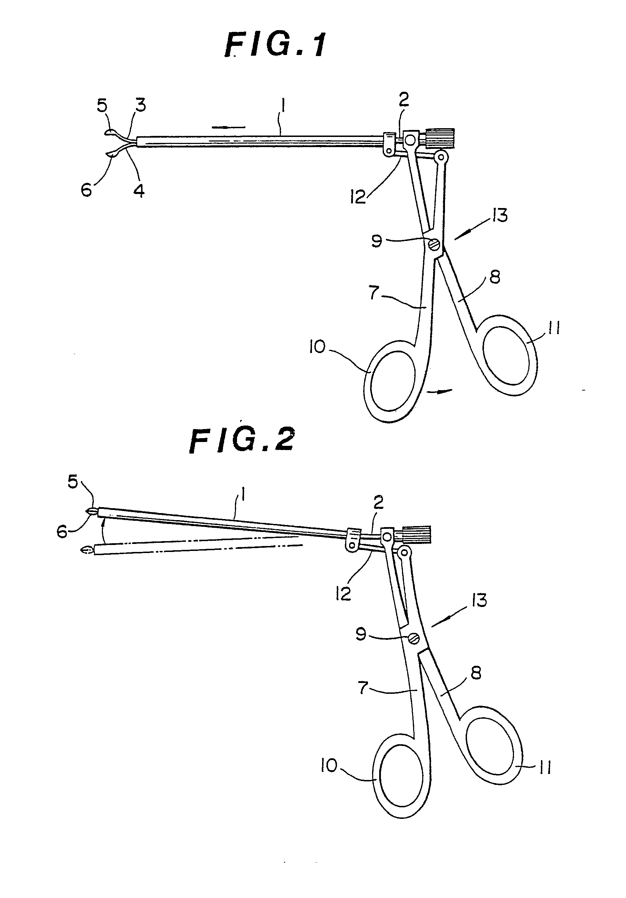

Before describing the present invention, a known biopsic forceps will be explained in detail with reference to Figures 1 and 2. A shaft 2 slidably inserted through a pipe 1, has at its tip a pair of resilient holding pieces which separate to form a V, being separately or integrally fixed to the end of this shaft 2, and intermeshing cup-shaped cutting blades 5 and 6 are provided respectively at the open ends of these holding pieces 3 and 4. A handle 7 of an operating part 13 is pivoted through a link 12 to the rear end of the pipe 1 and a handle 8 is pivoted to the rear of the shaft 2. These handles 7 and 8 intersect with each other at a fulcrum 9, and have respective finger grips 10 and 11 at the bases, so that operation to open or close this pair of handles 7 and 8, will cause the shaft 2 to advance or retreat through the pipe 1, and thereby the holding pieces 3 and 4 will project from or be withdrawn into the pipe 1, so as to open or close. In the thus formed biopsic forceps, when the finger grips 10 and 11 of the pair of handles 7 and 8 are operated to open the grips of the handles 7 and 8 and the shaft 2 slides forward (leftward in the drawing) in the pipe 1 to project out as shown in Figure 1, so that the holding pieces 3 and 4 separate to form a V due to the resiliency. In this state, when the biopsic forceps is operated to grasp a structural specimen of an affected part within a living body cavity in the cup-shaped cutting blades 5 and 6, the finger grips 10 and 11 of the handles 7 and 8 are then operated to move together to close the handles 7 and 8, the shaft 2 slides rearward (rightward in the drawing) so that the cutting blades 5 and 6 will be meshed with each other to cut off a structural specimen of the affected part into the cutting blades 5 and 6.

However, in this conventional biopsic forceps, in the event that the structure of the affected part is hard, then if the handles 7 and 8 have to be strongly urged together the link 12 will strongly press against the pipe 1, which link 12 may be pushed up, while describing an arc upward due to the pivoting point of the pipe 1 and link 12 as a fulcrum as shown in Figure 2, so that as a result the shaft inserted through the pipe 1 is also pushed upward and the pipe 1, shaft 2 and link 12 subjected to an unreasonable force in the bending direction, and may be bent or broken.

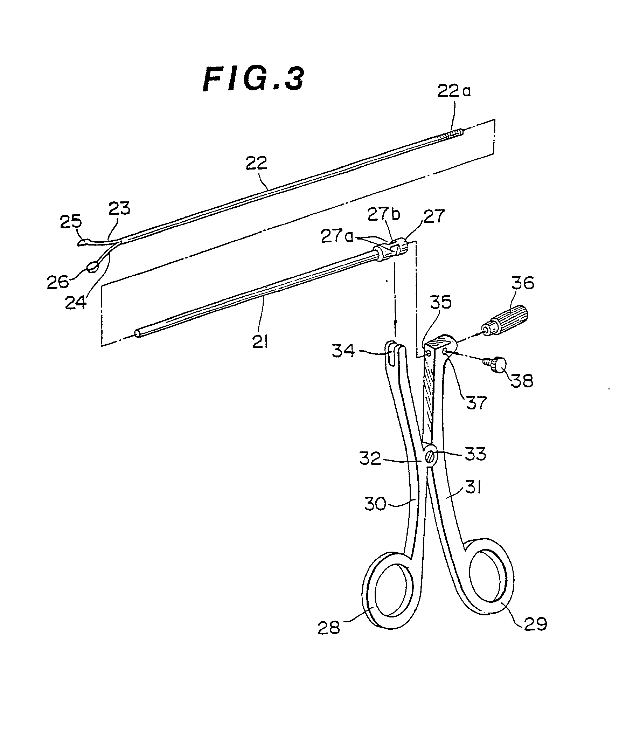

The embodiment of the present invention shown in Figures 3 to 5 has a pipe 21 slidably enclosing the shaft 22 having a screw thread 22a at the rear end. A pair of resilient holding pieces 23 and 24 to separate and open in the form of a V when projecting from the pipe are separately or integrally fixed to the tip of this shaft. Respective cup-shaped cutting blades 25 and 26 are formed at the open ends of the holding pieces 23 and 24. A tubular base 27 is fixed to the rear end of the pipe 21 and has incised surfaces 27a formed on opposed side surfaces as an engaging part 27b. A pair of handles 30 and 31 having respective finger grips 28 and 29 are pivoted by an off-set pivoting pin 33 at the centre 32 so that the handles do not need to intersect each other, so avoiding any weakened section. When the finger grips 28 and 29 are opened, the pair of handles close. A U-shaped groove 34 is formed at the tip of the front handle 30 so as to be slidably engaged with the engaging part 27b formed at the rear 27 of the pipe 21 and support the pipe 21. A through bore 35 houses the screw thread 22a of the shaft 22, which passes to the rear side of the tip of the rear handle 31. A nut 36 is screwed to the screw 22a that projects rearwardly out of the through bore 35, so that the shaft 22 may not be pulled out of the handle 31. Further, a screw hole 37 intersecting at right angles with the through bore 35 is provided on the front of the handle 31. A fixing screw 38 is screwed in this screw hole 27 so as to make the forward and rearward position of the shaft 22 in the through bore 35 adjustable. After adjustment, the fixing screw 38 secures the shaft 22 fixedly to the handle 31.

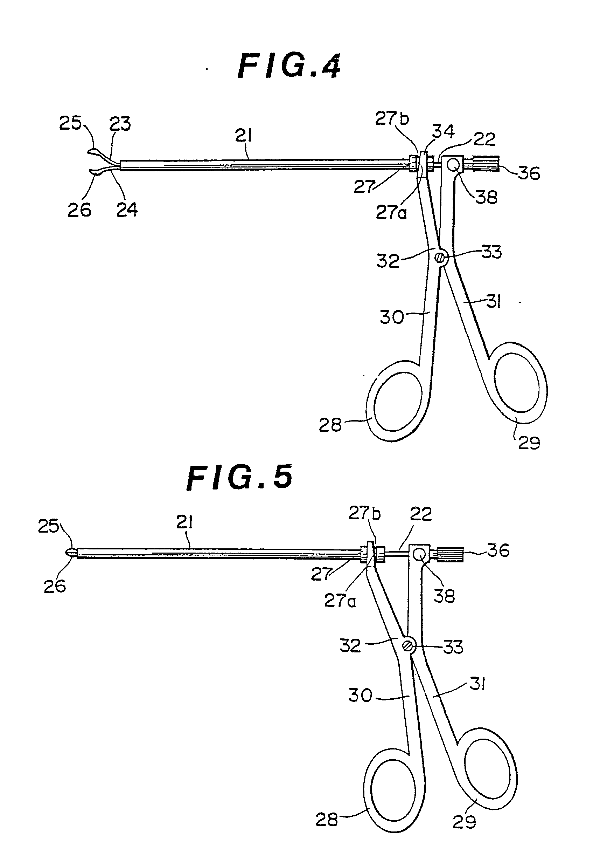

In the thus formed biopsic forceps, when the finger grips 28 and 29 of the respective handles 30 and 31 are operated in the opening direction, the tips of the handles 30 and 31 will move together, the pipe 21 will be moved to the right (in Figure 4) and the holding pieces 23 and 24 of the shaft 22 will project from the pipe 21 so as to separate and open in the form of a V due to the resiliency, as shown in Figure 4. In this state, when the biopsic forceps is operated to contain the structure of an affected part within a living body cavity into the cutting blades 25 and 26 and then the finger grips 28 and 29 of the handles 30 and 31 are pressed together to cause the tips of the handles 30 and 31 to move apart, unlike the arrangement shown in Figures 1 and 2, the pipe 21 will be moved to the left in Figure 5, and the cutting blades 25 and 26 will be meshed with each other, as shown in Figure 5, to cut off the affected part structural specimen. As the pipe 21 is always moved to right or left in a straight axial line direction along the shaft 22, even if the affected part structure is hard, and a very strong force has to be applied to the pipe 21, the pipe 21 will not be pressed upward, and therefore the shaft 22 will not be subjected to any unreasonable bending force, and is not liable to be either bent nor broken.

**Claim**

A biopsic foceps having a shaft (22) on which a pair of elongate resilient holding pieces (23, 24) are provided at the tip so as to separate and open in the form of V from each other when caused to project from a pipe (21) in which said shaft is slidably fitted, mutually opposed cutting blades (25, 26) being provided at the respective free ends of the pair of holding pieces so that, when the holding pieces project out of the pipe said pair of holding pieces separate the cutting blades and when the holding pieces are retracted into the pipe said pair of holding pieces close to mesh the cutting blades with each other, said shaft being supported at the rear of said pipe by the end of one of a pair of handles (30, 31) having respective finger grips (28, 29) and pivoted to open and close about a central fulcrum, said pipe being supported at the rear by the end of the other of said pair of handles, so that when the handles are operated said pipe moves axially forward or rearward on a stright line defined by the axis of said shaft, said pair of handles being pivoted by an off-set pin (33) so that when the finger grips are pushed apart said ends of the handles come together, characterised in that the shaft (22) is adjustably clamped at the rear of the pipe to the ends of its handle (31) by an off-set clamping means (38) and that the pipe (21) is slidably supported in a U-shaped groove (34) at the end of its handle (30) by an engaging part (27b) of a tube (27) provided at the end of the pipe and having incised surfaces (27a) to positively locate the pipe axially with respect to the end of its handle whilst permitting freedom of movement in the direction of the longitudinal axis of the handle.

## Patentanspruch

Eine Biopsiezange, die einen Schaft (22) hat, woran ein Paar langgestreckter, biegsamer Haltestücke (23, 24) an der Spitze angebracht sind, so dass sie sich in V-Form voneinander trennen können, wenn sie aus der Röhre (21) ausgeführt werden, in welche der Schaft gleitbar eingepasst ist, sich gegenüberliegende Schneideklingen (25, 26), die an den freien Enden eines Paares von Haltestücken so angebracht sind, dass beim Ausführen der Haltestücke aus der Röhre das genannte Paar von Haltestücken die Schneideklingen trennt, und wenn die Haltestücke in die Röhre eingezogen sind, so schliesst das genannte Paar von Haltestücken dicht, um die Schneideklingen ineinandergreifen zu lassen, wobei der genannte Schaft am hinteren Teil der Röhre vom Ende eines Paares von Handgriffen (30, 31) getragen wird, die entsprechende Fingergriffe (28, 29) haben und gelenkig verbunden sind zur Drehung um einen zentralen Drehpunkt, wobei die genannte Röhre hinten durch das Ende des anderen Paares von Handgriffen getragen wird, so dass bei Betätigung der Handgriffe die genannte Röhre sich axial vorwärts oder rückwärts entlang einer geraden Linie bewegt, die durch die Achse des genannten Schafts bestimmt wird, das genannte Paar von Handgriffen durch einen versetzten Stift (33) drehbar gelagert ist, so dass beim Auseinanderdrücken der Fingergriffe die genannten anderen Enden der Handgriffe zusammenkommen, dadurch gekennzeichnet, dass der Schaft (22) angleichbar am Ende der Röhre bis zum Ende seines Handgriffes (31) durch ein versetztes Klemmmittel (38) angeklemmt ist und das die Röhre (21) gleitbar getragen wird in einer U-förmigen Nut (34) am Ende Ihres Handgriffes (30) durch einen Eingriffteil (27 b) einer Röhre (27), vorgesehen am Ende der Röhre, und eingeschnittene Oberflächen (27 a) aufweisend, um die Röhre zwangsläufig axial festzulegen in bezug auf das Ende ihres Handgriffes, während eine freie Bewegbarkeit in Richtung der Längsachse des Handgriffs ermöglicht wird.

## Revendication

Pince à biopsie comprenant une tige (22) sur laquelle deux éléments de retenue élastiques et allongés (23, 24) sont montés à la pointe de manière à se séparer à et s'ouvrir en forme de V l'un par rapport à l'autre lorsque les amène à faire saillie d'un tuyau (21) dans lequel ladite tige est passée de façon coulissante, des lames de coupe (25, 26) mutuellement opposées étant prévues aux extrémités libres respectives des deux éléments de retenue de manière que lorsque ces derniers font saillie du tuyau, lesdits deux élément de retenue séparent les lames de coupe, et que lorsque les éléments de retenue sont rétractés dans le tuyau, lesdits deux éléments de retenue se referment pour faire coopérer les lames de coupe l'une avec l'autre, ladite tige étant supportée à l'arrière dudit tuyau par l'extrémité de l'une des deux poignées (30, 31) présentant des parties de saisie (28, 29) respectives et montées de façon pivotante de manière à s'ouvrir et à se fermer autour d'un point de pivotement, ledit tuyau étant supporté à l'arrière par l'extrémité de l'autre desdites deux poignées de manière que lorsque les poignées sont actionnées ledit tuyau se déplace axialement vers l'avant et vers l'arrière le long d'une ligne droite définie par l'axe de ladite tige, les deux poignées étant montées de façon pivotante sur un axe décalé de manière que lorsque les parties de saisie sont écartées, les extrémités des poignées se rapprochent, caractérisée en ce que ladite tige (22) est serrée de façon réglable à l'arrière du tuyau à l'extrémité de sa poignée (31) par des moyens de serrage décalés (38) et que le tuyau (21) est supporté de façon coulissante dans une gorge en forme de U (34) à l'extrémité de sa poignée (30) par une partie d'engagement (27b) d'un tube (27) prévu à l'extrémité du tuyau et présentant des surfaces découpées (27a) pour positionner positivement le tuyau axialement par rapport à l'extrémité de sa poignée tout en autorisant une liberté de mouvement en direction de l'axe longitudinal de la poignée.

0 0 6 6 4 6 5

# FIG.1

# FIG.2

1

# FIG.3

0 066 465

# FIG.4

# FIG.5